# EUROPEAN PATENT APPLICATION

(11) **EP 4 480 848 A1**
(43) Date of publication of application: **25.12.2024**
(21) Application number: 23180746.2
(22) Date of filing: 21.06.2023
(51) Int. Cl.: B65D 81/20, B65D 81/00, B01L 3/00, A61B 5/145, B01L 1/00

(54) **A PACKAGING ASSEMBLY FOR A SENSOR CALIBRATION SYSTEM AND A USE OF A PROTECTIVE GAS IN A PACKAGING ASSEMBLY**

(71) Applicant: SenTec AG, 4106 Therwil (CH)
(72) Inventor: Schumacher, Peter Matthias, 3038 Kirchlindach (CH); Stockbauer, Stefan, 79588 Efringen-Kirchen (DE)
(74) Representative: Hepp Wenger Ryffel AG

(57) **Abstract**

A packaging assembly (10) has a gas cartridge (2) with a calibration gas (CG) for calibrating a sensor. The gas cartridge (20) has an opening (22) configured to allow passage of gas, and at least one seal (24, 24a; 25) at or around the opening (22) for sealing the opening. The calibration gas (CG) is formed by a mixture of gas with at least one first component having a permeation rate through the at least one seal (24, 24a; 25) different from the permeation rate of another component of the calibration gas (CG). The assembly further has an envelope (30) having at least one wall defining an inner sealed space (31) between an inner surface of the wall and an outer surface or the gas cartridge. The at least one seal (24, 24a; 25) is arranged within the inner space (31). The inner space (31) includes a protective gas (PG) for counteracting the permeation of at least the first component of the calibration gas with a counterpressure.

## Description

The present invention relates to a packaging assembly for a sensor calibration system and a use of a protective gas in a packaging assembly according to the preamble of the independent claims.

Transcutaneous monitoring of gas (in particular CO2 or O2) provides clinicians with insight to identify trends and assess patient status.

Currently, transcutaneous gas is primarily measured using electrochemical skin sensors, which require regular calibration to ensure or increase their accuracy.

Calibration of the sensors is performed by testing the sensor by using a calibration system generally having a gas cartridge containing a mixture of gases with a predefined percentage of the respective gases.

Cartridges used in calibration systems need to have a constantly precise concentration in gases to allow efficient calibration of sensors and thus to ensure the measurements obtained by the sensors are correct.

The cartridges are provided with seals generally made of materials that are substantially gas-tight and thus stop the passage of gases. However, the sealing materials commonly used are not entirely gas impermeable and have different permeation rates depending on the material and the gases.

Permeation of gases from the cartridge at different rates hence leads to a change or an unbalance in the gas mixture within the gas bottles over time. A change in the ratio of the gases within the cartridge leads to a deviation in the calibration of sensors because the calibration is not carried out against the assumed predefined percentage, i.e. the concentration the gas cylinder should have if there were no permeation leakage.

The gradually increasing imbalance in the gas mixture over time thus defines the shelf-life of the gas cartridges. Especially with small volume cartridges having a smaller ratio between the volume and the sealing surface, the effect of the difference in permeation becomes more prominent and the shelf life is reduced.

It is an object of the present invention to overcome the disadvantages of the prior art and in particular to provide a packaging assembly with at least one gas cartridge comprising a calibration gas which has a long shelf life, in particular if the volume of the gas cartridge is relatively small.

Sealed packagings are commonly used in the various industries such as the food industry to prolong the shelf-life of consumable goods and in the medical field to ensure medical instruments remain sterile and limit the risk of contamination of the instrument and also to protect the medical instrument during shipping.

Various techniques of packaging have been developed over the years to protect the content of the packaging from external factors such as oxygen, water vapor, light, pathogens amongst others.

EP 3995411 A1 relates to a packaging for a medical device in view of the transportation of the device. The packaging comprises header bag comprising a sheet configured to be sealed to form an enclosure for the medical device. The sheet of the packaging comprises an inner layer and an outer layer. The inner layer is impervious to gas so that a vacuum can be created between the inner and outer layers. The packaging serves to ensure that the medical device is not contaminated during transportation.

WO 2019009868A1 discloses a vacuum packaging for a food product. The packaging comprises two walls: an upper cover and a lower backing. The walls are fastened to one another in an airtight manner along a peripheral line that is positioned on the edge of the packaging.

EP3959155A1 pertains to a food package designed to improved long-term quality of the food product. The packaging comprises a heat-sealed cover having a lower plastic film and an upper plastic film. The upper and lower film are collapsible and heat-sealed to each other at perimeter edges to define a closed, watertight internal housing filled with a modified atmosphere. The packaging further comprises a tray inserted in the closed housing.

The above object is solved with a packaging assembly and a use according to the characterizing portion of the independent claim.

The packaging assembly comprises at least one gas cartridge comprising a calibration gas for calibrating a sensor. The gas cartridge has an opening configured to allow passage of gas and at least one seal at or around the opening for sealing the opening.

Typically, there might be two types of seals, a first seal sealing a closure of the cartridge against a body of the cartridge and a second seal arranged in a valve placed in the closure and allowing an exit of the gas during use.

The seals may be made of any sealing material that is at least partly gas-tight. For example, the seal is generally made of an elastomer or rubber material which may include silicon; EPDM; nitrile; neoprene; FPM/FKM; butyl. Alternatively, the seal may be a mixture of silicone; EPDM; nitrile.

The calibration gas is formed by a mixture of gas with at least one first component having a permeation rate through the at least one seal different from the permeation rate of another component of the calibration gas, if the at least one seal is sealing against the atmosphere. Typically, plurality of components may have different permeation rates compared to one another.

The calibration gas is generally a mixture of gases to be used for the calibration of medical sensors such as transcutaneous sensors. For example, the calibration gas may be any gas mixture adapted for use in transcutaneous CO2 sensors.

The assembly further comprises an envelope having at least one gas impermeable wall. The at least one wall defines an inner sealed space between an inner surface of the wall and an outer surface or the gas cartridge. The at least one seal of the gas cartridge is arranged within the inner space of the envelope. The inner space includes a protective gas for counteracting the permeation of at least the first component of the calibration gas with a counterpressure.

The envelope can have any size, shape, material or rigidity as long as the inner sealed space can at least contain the at least one seal.

The envelope may be made of one continuous gas impermeable wall. The continuous wall may at least partly fold on itself so as to form the envelope. Alternatively, the envelope may comprise a plurality of gas impermeable walls. For example, the plurality of walls may be connected to one another thus forming the envelope. Preferably, the plurality of walls are connected in a gas-tight manner. Optionally the plurality of walls may be sealed together in the same manner that the envelope is sealed.

The wall(s) of the envelope has/have an inner and outer surface. An inner space of the envelope is delimited between an inner surface of the envelope wall(s) and an outer surface of a gas cartridge. The inner space of the envelope is configured to contain the protective gas.

In this context, gas-tight and gas impermeable and highly gas-impermeable are understood as meaning that substantially no amount, or a small or an insignificant amount, of gas is able to pass through the material of the envelope.

The gas-impermeable wall of the envelope maintains the protective gas within the inner space of the envelope and thus, prevent the protective gas from leaking.

The protective gas is a gas or mixture of gases chosen such as to at least partly counteract the permeation of the components of the calibration gas through the at least one seal of the gas cartridge. Thus, in presence of and due to the protective gas, the permeation rate of at least one component of the calibration gas may be counteracted more than the permeation rate of another component. The presence of the protective gas therefore, helps to maintain the calibration gas at the assumed concentration that is necessary for efficient calibration of sensors for example.

The counteracting protective gas mitigates the difference in permeation rates of the components of the calibration gas trough the seal(s) of the cartridge and hence helps prolong the shelf-life of the gas cartridge.

As permeation works through diffusion, the permeating gas component, moves across the seal from the internal environment within the cartridge having a high concentration of the component to the external environment having a low concentration of the component (i.e. outside the gas cartridge).

The permeation rate of a gas is hence driven by differences in partial pressures and the material properties of the sealing material. The invention is based on that finding that the pressure gradient between the internal and external partial gas pressures can be influenced by the provision of an appropriate protective gas.

Taking the example of a gas cartridge having a calibration gas including CO2 and O2, if the gas cartridge has an 8% CO2 content, the external partial pressure of CO2 can be matched with the internal partial pressure of CO2. The partial pressure of one component influences the partial pressure of other components. Therefore, to obtain an acceptable shelf-life of the gas cartridge, the partial pressure of all the components is preferably be considered.

Therefore, for example, even with a 13% CO2 content of the calibration gas, (which would be at 1.43 bar partial pressure) a packaging with 0.8 to 1 bar CO2 of protective gas could significantly increase shelf life.

For example, an ordinary gas cartridge having a shelf life of 12 months, may, when provided with the packaging assembly have a shelf-life of about 24 months. Advantageously, the shelf-life of the gas cartridge may be improved by a factor of up to 10, depending on the impermeability of the material of the envelope.

The gas cartridge can be any container adapted to hold gas such as for example bottles, tanks, cylinders, canisters, etc.

In a preferred embodiment, the envelope encompasses the whole gas cartridge. In other words, the entire gas cartridge is encompassed by the envelope and entirely situated within the inner space defined by the wall(s) of the envelope.

Alternatively, only part of the gas cartridge can be encompassed by the envelope. For example, the envelope may encompass the opening of the cartridge or partly encompass the walls of the cartridge.

The envelope may then be sealingly attached or attachable to an outer surface of the gas cartridge.

The packaging assembly may comprise a plurality of gas cartridges and the envelope is configured to attach or encompass at least partly or wholly the plurality of gas cartridges.

The envelope may have a wall having a thickness from about 40pm to 400pm if it is intended to remain flexible. Envelopes where a certain stiffness is acceptable under usability aspects may have a thickness of up to 3000pm.

The permeation rate through the walls of the envelope linearly scales with thickness of a given material. The thickness of the wall(s) of the envelope may hence be chosen in dependence of the material and in such a manner that the permeation rates of the components of the protective gas through the envelope are sufficiently low and preferably sufficiently similar.

The envelope may include or be made of a plastic material and/or a metal material. In particular, the plastic material or metal material can be chosen to be highly impermeable to CO2, O2, N2 and/or water vapor (H2O)). The plastic material is preferably selected from the group of EVOH, PVDC, PE, PP, PET, a combination of PA/PE, a combination of PET/PE. The metal material is preferably selected from steel, tin, aluminium, a combination of steel, tin, aluminium.

Additionally or alternatively, the envelope may include a foil. The envelope, optionally the foil material, may be coated or layered with a coating material adapted to reduce the permeation rates of gases through the material of the envelope. For example, the coating or layered material may include aluminium, silver, silicon oxide, aluminium oxide. The aluminium and/or silver used in the coating material generally provide good barrier properties against gas and moisture whereas silicon oxide and/or aluminium oxide used in the coating material generally provide resistance to oxygen or water vapor.

The envelope may be formed as a sealed bag. Alternatively, the envelope may be formed as a sealed can. The sealed bag or the sealed can is preferably dimensioned so as to fully encompass the gas cartridge or gas cartridges. Alternatively, the envelope can be dimensioned so as to encompass only a portion of the gas cartridge(s). For example, the envelope may be shaped and dimensioned in a cap-like manner, so as to encompass only the area neighbouring the opening of one or a plurality of gas cartridges.

The envelope may additionally comprise an opening element configured to facilitate the opening of the envelope.

The opening element is configured to provide an easy, time-efficient means for opening the envelope without having to use tools. The opening element may be, for example, a tear-tab formed by a portion of the sealed bag and adapted to be easily torn away to open the bag. For a can, the opening element may be a pull tab on a lid of the can and configured to allow the lid to be peeled away.

In some embodiments, the protective gas and the calibration gas have at least partly the same components.

At least one component of the protective gas and of the calibration gas are the same component. Alternatively, a plurality, or all, of the components of the protective gas and of the calibration gas are the same.

Including the same gas component(s) in the protective gas and in the calibration gas may help ensure that the protective gas and calibration gas permeate through the seal at the same rate.

The calibration gas and/or the protective gas includes one or more of CO2; O2. The calibration gas may further include an inert gas. The inert gas may be any suitable inert gas such as Nitrogen; Argon; Neon.

The calibration gas may therefore further include N2, Ar.

The calibration gas may, optionally, include at least two of CO2; O2; N2. Further optionally, the calibration gas may include all of CO2; O2; N2.

In some embodiments the protective gas comprises CO2 and/or O2.

The protective gas can be a mixture of CO2 and O2, i.e. excluding any other components. The protective gas may comprise more CO2 than O2 by weight.

The gas cartridge can have a filling pressure between 3 to 18 bar, preferably between 6 to 12 bar, and more preferably of between 9 to 10 bar. Typically, the gas cartridge has a filling pressure of approximately 10 bar.

The filling pressure of a gas cartridge corresponds to the maximum gas pressure in the cylinder against ambient pressure upon completion of filing the gas cartridge with a gas.

For a gas cartridge (including 8% CO2, 12% O2, 80% N2 by volume) with 10 bar filling pressure according to the prior art, the difference in partial pressures of the gas components are 0.88 bar CO2, 1.11 bar O2 and 8.01 bar N2 compared to air at ambient pressure at sea level. These partial pressure differences determine the permeation rates in addition to the properties of the material of the seals and their geometries. The permeation of CO2 is much higher than for the other components in most polymeric sealing materials. Using a packaging assembly according to the invention and having protective gas with a 80% CO2, 20% O2 mixture at ambient pressure, results in partial pressure differences of 0.08 bar CO2, 1.12 bar O2 and 8.80 bar N2. The driving partial pressure difference for CO2 is thus 10 times lower. Therewith, the difference in permeation rates is substantially reduced.

The components of the protective gas and/or of the calibration gas can be selected such that in the presence of the protective gas, the difference between the permeation rates of the components of the calibration gas is reduced, in particular such that the permeation rate of CO2 and O2 of the calibration gas through the seals is substantially the same.

If the permeation rate scaled with the percentage gases in the mixture, the ratio of the gases within of the mixture would not be influenced over time. As explained above, according to the invention, the difference in permeation coefficient of the material is compensated with a corresponding difference in partial pressure.

In a two gas mixture this is possible in most configurations.

In a gas mixture having three or more gases, a full compensation may not be possible in all situations, especially considering restrictions in view of safety. In this case, the difference between the rates is preferably reduced as much as possible under practical or safety consideration.

In particular, for a defined calibration gas (CG), the components of the protective gas (PG) are selected such that, in the presence of the protective gas (PG), the percentage of the components of the calibration gas (CG) stays within a 0.1% range, preferably 0.075% or lower range, of the intended concentrations during the intended shelf life.

In a predetermined system, e.g. 0.5l gas cartridge with a calibration gas comprising 8% CO2, 12% O2 and 80% N2, the protective gas is chosen in a way that, the change of the percentage of CO2 and O2 of the calibration gas due to permeation through the seals, remains less than 0.075% for a period of at least 12 months.

Preferably, the above-mentioned components of the protective gas and/or the calibration gas sum up to 100%. The balance is brought to 100% by adding the inert gas to predefined percentages of CO2 and O2. Preferably, the components of the calibration gas include 3-13% CO2; 6-21% O2; 66-91% N2. Further optionally, the components of the protective gas include 25-90% CO2; 10-50% O2.

More preferably, the calibration gas may comprise 7.5-8% CO2; 12-21% O2; 71.0-80.5% N2. Typically, the calibration gas comprises:
- 8% CO2, 12% O2 and 80% N2;
- 7.5% CO2, 20.9% O2, 71.6% N2;
- 7.5% CO2, 12% O2, 80.5% N2.

Typically, the protective gas comprises 21% O2 and 79% CO2.

Preferably, the gas cartridge has a volume of less than 0.6l.

Preferably, the gas cartridge has a volume of about 0.2 - 0.5l.

The protective effect of the packaging assembly may be inversely correlated with the volume of the gas cartridge. Thus, the smaller the volume of the gas cartridge, the more the protective effect of the packaging may be significant.

The packaging assembly may additionally have an indicator within the envelope for indicating if the packaging is functional.

The indicator may be a CO2 indicator capable indicating a change in partial pressure of CO2 in the protective gas. The indicator can be a printable indicator adapted to change colour when the partial pressure of CO2 in the protective gas drops below a certain value, e.g. through leakage or permeation.

Alternatively, the indicator may detect humidity and be adapted to detect a breach in the packaging assembly. The indicator can be a printable indicator adapted to change colour depending on humidity levels. In cases where the packaging assembly were damaged, e.g. a tear in the envelope, the protective gas would equilibrate with the ambient air generally having more than 20% relative humidity and the indicator would change colour.

Providing an indicator allows the user to confirm that the calibration gas still has the correct ratio of its components even after the extended shelf-life and, thus, that the calibration gas is suitable for its intended use. In this context it may be advantageous to make at least a part the envelope of a transparent material.

In a second aspect, the invention pertains to the use of a protective gas in a packaging assembly to reduce the difference in permeation rates of components of a calibration gas having different partial pressure though a seal of a gas cartridge comprised within the packaging assembly, wherein the protective gas and the calibration gas have at least partly the same components.

The packaging assembly may be a packaging assembly such as described above.

The calibration gas for use in the packaging assembly includes one or more of CO2; O2 or is a mixture of CO2 and O2. The calibration gas may further include an inert gas, such as for example N2.

### Brief description of drawings:

- Fig. 1: is a schematic cross-section view of a first embodiment of a packaging assembly according to the invention.
- Fig. 2: is a schematic cross-section view of a second embodiment of a packaging assembly according to the invention.
- Fig. 3: is a schematic cross-section view of a packaging assembly comprising a plurality of gas cartridges.
- Fig. 4: is a schematic cross-section view of an embodiment of a valve for a gas cartridge according to the invention.

### Details Description of preferred embodiments:

Non-limiting embodiments of the invention are now described by way of example only.

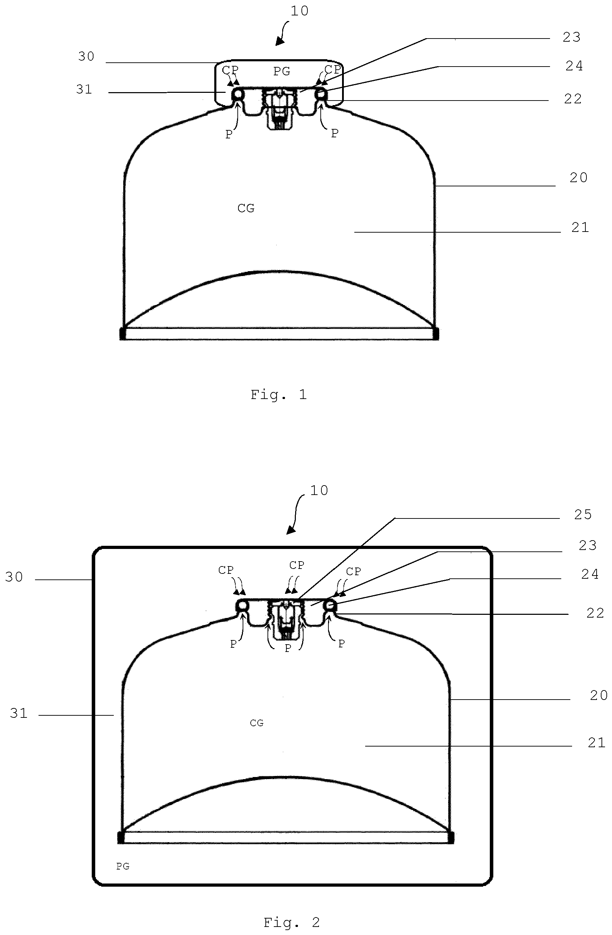

Fig. 1 shows a packaging assembly 10 comprising a gas cartridge 20 and an envelope 30.

The gas cartridge 20 comprises an interior surface and an exterior surface. The interior surface defines a chamber 21 containing the calibration gas (CG).

The gas cartridge 20 comprises a circular rim on its surface forming an opening 22. The opening 22 is adapted to allow passage of gas from within the gas cartridge 20 to outside the gas cartridge. The gas cartridge 20 comprises a valve 23 attached by crimping to the rim in the gas cartridge. The valve 23 allows controlled dispersion of gas from within the gas cartridge 20 to outside the gas cartridge in a manner known to the skilled person, for example, to allow controlled usage of the calibration gas to calibrate medical sensors such as transcutaneous sensors.

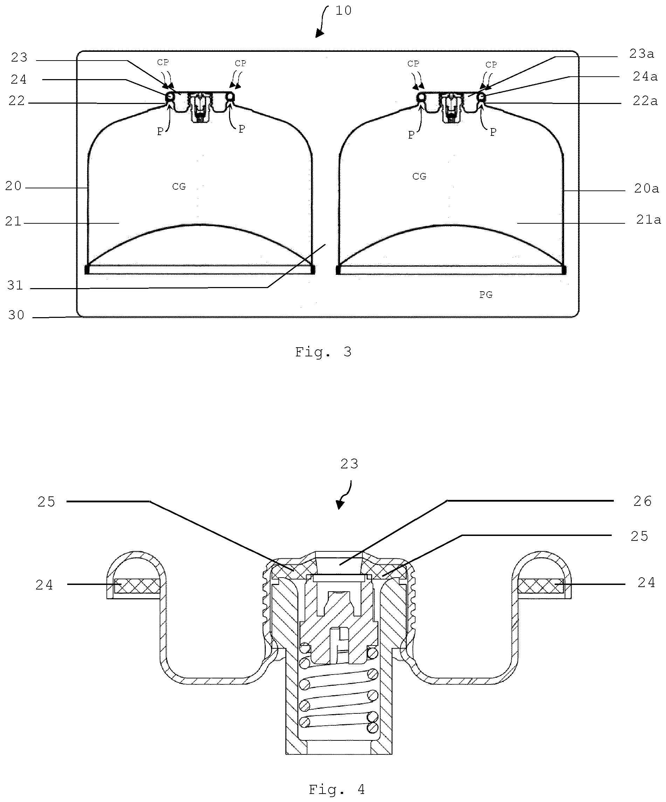

The gas cartridge 20 comprises a seal 24 around the opening 22 and situated between the gas cartridge 20 and the valve 23. The valve comprises a second seal (see Fig. 4). The seal 24 serves to prevent or reduce leakage of the calibration gas (CG) from chamber 21 of the gas cartridge 20.

The envelope 30 comprises four walls delimiting an inner space 31. The inner space 31 is formed by the free space between the wall(s) of the envelope 30 and the outer surface of the wall(s) of the gas cartridge 20.

The envelope 30 is positioned so that it encompasses the protrusion of gas cartridge and thus also encompasses the opening 22 and the seal 24 of the gas cartridge 20. Further, it is sealingly contacting the outer surface of the cartridge 20, e.g. by gluing or welding, such as to securely close the inner space 31.

A protective gas (PG) at ambient pressure is arranged within the envelope 30 and applies a gas specific counter pressure (CP) to the seal 24 and thus counter acts the permeation (P) of the calibration gas (CG) through the seal 24.

In a specific embodiment, the protective gas PG and the calibration gas CG of the specific embodiment are chosen as follows, for a cartridge with a volume of 0.3 l and a filling pressure of 10 bar and with a volume of the inner space 31 of 0.05l to 2.0l:

| | **CG** | **PG** |
|---|---|---|
| O2 | 12 % | 21 % |
| CO2 | 8 % | 79 % |
| N2 | 80% | 0 % |

As shown in Fig. 2, the packaging assembly 10 is the generally the same as in Fig.1, the only difference being that the envelope 30 encompasses the entire gas cartridge 20 rather than just a portion of the gas cartridge 20 as in Fig. 1. Same reference numbers designate the same parts. Additionally, the valve of Fig.2 comprises an additional seal 25.

The envelope 30 comprises two gas-impermeable walls delimiting an inner space 31. The inner space 31 is formed by the free space between the wall(s) of the envelope 30 and the outer surface of the wall(s) of the gas cartridge 20. The walls are designed so as to be able to encompass the entire gas cartridge 20 and are gas-tight to avoid leakage of the protective gas (PG). The walls are made of two substantially rectangular foils of a plastic materials which are sealed together along their periphery, e.g. by ultrasonic welding, in a manner known for packaging materials. Prior to final sealing, the inner space 31 is filled with the protective gas. The plastic material in a specific embodiment is a laminated plastic foil of PE, PET and aluminium with a thickness of 91µm. The layers of the plastic foil are generally disposed so that the layer of aluminium is sandwiched between the layer of PE and the layer of PET. More specifically, in this embodiment, the outermost layer of the plastic foil is PET with a thickness of 12µm and the innermost layer is PE with a thickness of 70pm. The aluminium layer having a thickness of 9µm.

The protective gas (PG) at ambient pressure of the envelope 30 applies a gas specific counter pressure (CP) to the seal24 of the gas cartridge 20 and to the second seal 25 of the valve 23 and thus counteracts the permeation (P) of the calibration gas (CG) through both the seal 24 at the opening 22 of the gas cartridge 20 and the second seal 25 of the valve 23.

Fig. 3 depicts a packaging assembly 10 having two gas cartridges 20 and 20a. The envelope of the packaging assembly fully encompasses both of the gas cartridges 20, 20a.

Both of the gas cartridges 20, 20a have openings 22, 22a adapted to allow passage of a calibration gas (CG) from chambers 21, 21a of the gas cartridges 20, 20a. They further comprise valves 23, 23a for controlling the passage of gas and seals 24, 24a for preventing leakage of the calibration gas (CG) from the chambers 21, 21a.

The envelope 30 comprises gas-impermeable walls delimiting an inner space 31. The inner space 31 is formed by the free space between the wall(s) of the envelope 30 and the outer surfaces of the wall(s) of the gas cartridges 20, 20a. The walls are designed so as to be able to fully encompass the two gas cartridges 20, 20a and to be gas impermeable.

Fig. 4 shows a closer view of the valve of Fig. 2. The valve is adapted to be attached to the gas cartridge 20, e.g. by crimping. The valve has a first seal 24 to be positioned at a circular rim forming an opening 22 of a gas cartridge 20. The first seal 24 prevents gas leakage at the connection point of the valve 23 to the gas cartridge 20. The valve further comprises a second seal 25 at a gas outlet 26 of the valve 23. The gas outlet 26 is configured to open to allow gas dispersion. The second seal 25 prevents gas leakage at the at the gas outlet 26 when the gas outlet is shut. The second seal 25 is made from an elastomer and is generally flat.

## Claims

1. A packaging assembly (10) comprising:
at least one gas cartridge (20) comprising a calibration gas (CG) for calibrating a sensor, the gas cartridge (20) having an opening (22) configured to allow passage of gas, and at least one seal (24; 24a; 25) at or around the opening (22) for sealing the opening,
wherein the calibration gas (CG) is formed by a mixture of gas with at least one first component having a permeation rate through the at least one seal (24; 24a; 25) different from the permeation rate of another component of the calibration gas (CG)
**characterized in that** the assembly (10) further comprises an envelope (30) having at least one wall defining an inner sealed space (31) between an inner surface of the wall and an outer surface or the gas cartridge (20), wherein the at least one seal (24; 24a; 25) is arranged within the inner space (31) and wherein the inner space (31) includes a protective gas (PG) for counteracting the permeation (P) of at least the first component of the calibration gas (CG) with a counterpressure (CP).

2. The assembly (10) according to claim 1, wherein the envelope (30) encompasses the whole gas cartridge (20).

3. The assembly (10) according to any claim 1 or 2, wherein the assembly (10) comprises a plurality of gas cartridges (20, 20a) and wherein the envelope (30) is configured to attach or encompass at least partly the plurality of gas cartridges (20, 20a).

4. The assembly (10) according to claim 1 to 3, wherein the envelope (30) is made of or includes a plastic material and/or metal material, in particular a plastic material and/or metal material which is highly impermeable to CO2, O2, N2 and/or water vapor (H2O)), the plastic material preferably selected from the group of EVOH, PVDC, PE, PP, PET, a combination of PA/PE, a combination of PET/PE, the metal material preferably selected from steel, tin, aluminium, a combination of steel, tin, aluminium.

5. The assembly (10) according to any claim 1 to 4, wherein the envelope (30) is formed as a sealed bag or a sealed can, optionally having an opening element configured to facilitate opening of the sealed bag or the sealed can.

6. The assembly (10) according to any claim 1 to 5, wherein the protective gas (PG) and the calibration gas (CG) have at least partly the same components.

7. The assembly (10) according to any claim 1 to 6, wherein of the calibration gas (CG) and/or the protective gas (PG) includes one or more of CO2; O2; an inert gas, preferably N2.

8. The assembly (10) according to any claim 1 to 6, wherein protective gas (PG) comprises CO2 and/or O2.

9. The assembly (10) according to claim 8, wherein the protective gas (PG) is a mixture of CO2 and O2.

10. The assembly (10) according to any claim 1 to 9, wherein the gas cartridge (20) has a filling pressure between 3 to 18 bar, more preferably between 6 to 12 bar and most preferably between 9 - 10 bar.

11. The assembly (10) according to claim 1 to 10, wherein the components of the protective gas (PG) and/or of the calibration gas (CG) are selected such that, in the presence of the protective gas, the difference between the permeation rates of the components of the calibration gas is reduced.

12. The assembly (10) according to claim 11, wherein the components of the protective gas (PG) are selected such that, in the presence of the protective gas (PG), the percentage of the components of the calibration gas (CG) stays within a 0.1% range, preferably 0.075% or smaller range, of the intended concentrations during the intended shelf life.

13. The assembly according to any claim 1 to 12, wherein the components of the protective gas (PG) and/or the calibration gas (CG) sum up to 100%, optionally wherein the components of the calibration gas (CG) include 3-13% CO2; 6-21% O2; 66-91% N2, further optionally, the components of the protective gas (PG) include 25-90% CO2; 10-50% O2.

14. The assembly (10) according to any claim 1 to 13, wherein the gas cartridge (20) has a volume of less than 0.6, preferably about 0.2 - 0.3L.

15. Use of a protective gas (PG) in a packaging assembly (10) to reduce the difference in permeation rates of components of a calibration gas (CG) having different partial pressure through a seal (24; 24a; 25) of a gas cartridge (20) comprised within the packaging assembly (10), wherein the protective gas (PG) and the calibration gas (CG) have at least partly the same components.

16. Use according to claim 15, wherein the calibration gas (CG) and/or the protective gas (PG) includes one or more of: CO2; O2; N2, optionally the protective gas (PG) being a mixture of CO2 and O2.
